# EUROPÄISCHE PATENTSCHRIFT

(11) **EP 1 183 029 B1**
(45) Veröffentlichungstag und Bekanntmachung des Hinweises auf die Patenterteilung: **27.07.2005**
(21) Anmeldenummer: 00929536.1
(22) Anmeldetag: 17.05.2000
(51) Int. Cl.: A61K 31/485, A61K 9/70, A61P 25/04, A61P 25/36

(54) **PHARMAZEUTISCHES PRÄPARAT ENTHALTEND DIAMORPHIN UND VITAMIN E SEINE VERWENDUNG ZUR BEHANDLUNG DER OPIATSUCHT**
PHARMACEUTICAL PREPARATION CONTAINING DIAMORPHINE AND VITAMIN E AND ITS UTILIZATION FOR TREATING OPIATE ADDICTION
PREPARATION PHARMACEUTIQUE CONTENANT DE LA DIAMORPHINE ET DE LA VITAMINE E ET SON UTILISATION POUR LE TRAITEMENT DE L'OPIOMANIE

(30) Priorität: 21.05.1999 DE 19923551
(43) Veröffentlichungstag der Anmeldung: 06.03.2002
(73) Patentinhaber: LTS Lohmann Therapie-Systeme AG, 56626 Andernach (DE)
(72) Erfinder: MATUSCH, Rudolf, D-35041 Marburg (DE); ADAM, Bernd, D--34613 Treysa (DE); KOCH, Andreas, D-56581 Melsbach (DE); HOFFMANN, Hans-Rainer, D-56566 Neuwied (DE); ASMUSSEN, Bodo, D-56170 Bendorf (DE)
(74) Vertreter: Schweitzer, Klaus, Dr.
(86) Internationale Anmeldenummer: PCT/EP2000/004458
(87) Internationale Veröffentlichungsnummer: WO 2000/071125

(56) Entgegenhaltungen:
- EP-A- 0 672 416
- WO-A-00/41683
- WO-A-92/13540
- WO-A-97/34587
- DE-A- 19 642 043

## Beschreibung

Die vorliegende Erfindung bezieht sich auf pharmazeutische Präparate zur Verwendung in einem Verfahren zur Behandlung von Opiatsucht bzw. der Opiatabhängigkeit, insbesondere der Heroinabhängigkeit. Der dabei verwendete Wirkstoff ist vorzugsweise Diamorphin (Heroin, Diacetylmorphin) und / oder eines seiner pharmazeutisch akzeptablen Säureadditionssalze.

Die Opiate sind Wirkstoffe des Schlafmohns (Papaver somniferum). Zu den wichtigsten Vertretern zählen Opium, Morphium, Kodein und Heroin. Auch Narkotin, Papaverin, Narcein, Thebain, Laudanosin, Xanthalin und Noscapin sind Inhaltsstoffe des Rohopiums. Es sind aber nur die Morphin-Alkaloide, von denen eine narkotisierende und schmerzstillende Wirkung ausgeht.

Bei häufigem Gebrauch von Opiaten, insbesondere bei Morphin-Alkaloiden wie Heroin, kommt es zu einer psychischen Gewöhnung (z. B. Flucht vor der Wirklichkeit des Alltags), zu der sich eine körperliche Gewöhnung dazugesellt. Bei Vorenthaltung der konsumierten Opiate treten typische Entzugserscheinungen, insbesondere starke Entzugsschmerzen auf. Die Opiatabhängigkeit bzw. Opiatsucht ist somit nicht mehr auf die vom Konsumenten im Anfangsstadium der Sucht erwünschte Euphorisierung bzw. Veränderung der Wahrnehmung zurückzuführen, sondern insbesondere auf die Vermeidung der bei Opiatunterversorgung auftretenden massiven Abstinenzschmerzen. Die neuerliche Opiatzufuhr führt dann zwar kurzfristig zum Erlöschen der Entzugsschmerzen, bei Daueranwendung von Opiaten treten aber neben körperlichem Abbau (fahles Aussehen, Schweissausbrüche bei geringstem Anlass, Magen-Darm-Störungen, Hautausschlag, Angina-pectoris-Anfälle, Störungen der Sexualsphäre mit Dys- und Amenorrhoe, Potenzminderung, Keimdrüsenschäden etc.) auch psychische Störungen (Reizbarkeit, Verstimmung, Depression etc.) und intellektuelle Fehlleistungen (Gedächtnisstörungen, Verlust der Konzentrationskraft, psychotische Zwangsvorstellungen etc.) auf. Dies führt den Süchtigen in eine Isolierung von dem früheren Bekanntenkreis, langfristig in einen sozialen Abstieg und nicht zuletzt auch in die Kriminalität.

Zur Behandlung von Heroinabhängigen wird seit einiger Zeit Methadon, ein vollsynthetisches Opioidanalgetikum eingesetzt. Dieses ist jedoch in der Therapie dem Heroin selbst bei intravenöser Applikation unterlegen, wie der seit 1994 in der Schweiz laufende und wissenschaftlich ausgewertete Versuch zur staatlich kontrollierten Heroinabgabe gezeigt hat. Dort wurde an 969 ausgewählte Heroinsüchtige, deren Krankengeschichte bereits mehrere erfolglose Therapieversuche und die gravierende, primär oder sekundär aus der Heroinsucht resultierende gesundheitliche Probleme aufwiesen, Heroin zur intravenösen Applikation oder zur Aufnahme über die Lunge mittels Zigaretten kontrolliert abgegeben. Die verabreichte Dosis konnte nach anfänglichem Anstieg (bedingt durch die beabsichtigt sehr geringe Anfangskonzentrationen), ab dem 6. Monat konstant gehalten, z.T. auch leicht gesenkt werden. Deutliche Verbesserungen am Gesundheitszustand der Heroinsüchtigen erzielt man durch staatlich kontrollierte Verabreichung von Heroin. Auch wird dabei ein signifikanter Rückgang der Beschaffungskriminalität durch Heroinsüchtige beobachtet. So betrug der gesamtvolkswirtschaftliche Nutzen durch Rückgang der Beschaffungskriminalität in der Schweiz 96 Franken pro Person und Tag.

Die intravenöse Verabreichung von Heroin hat jedoch starke Plasmaspiegelschwankungen zur Folge. So übersteigt die Konzentration von Heroin im Blutplasma kurz nach einer Injektion den minimal notwendigen Wert zur Unterdrückung der Entzugserscheinung stark und gelangt in den toxischen Bereich mit schweren Nebenwirkungen und Verwirrtheit. Durch Biotransformation sinkt der Plasmaspiegel bereits nach wenigen Stunden wieder auf eine Konzentration unterhalb der Wirkschwelle und es kommt zu den unerwünschten Entzugserscheinungen.

Die Verwendung von Buprenorphin zur Behandlung der Heroinabhängigkeit konnte auch noch nicht erfolgreich angewandt werden. Dies mag darauf zurückzuführen sein, dass Buprenorphin sehr teuer ist und als partieller Opiat-Agonist bei möglichen Überdosierungen grosse Probleme bereiten kann, weil z. B. hervorgerufene Atemdepressionen nicht durch Gabe eines normalerweise einzusetzenden Antagonisten therapierbar sind. Ausserdem zeigte sich auch bei längerem Einsatz von Buprenorphin, übrigens analog der Langzeittherapie von Methadon, eine Ausprägung zur Abhängigkeit.

Transdermale therapeutische Systeme zur Behandlung von Abhängigkeiten und / oder Suchtbekämpfung sind bekannt, jedoch haben diese bisher nur im Falle der Nikotinabhängigkeit zu kommerziell erhältlichen Produkten und gewissen Erfolgen der Therapie geführt. Vor allem bei Opiatabhängigkeit und Opiatmissbrauch, insbesondere Heroinmissbrauch haben sich die Hoffnungen in Lobelin oder Methadon nicht erfüllt.

Die WO 97 34587 A offenbart Heroin-enthaltende Transdermale Therapeutische Systeme (TTS) als Analgetika, die nur dann wirken, wenn die Epidermis entfernt ist.

In der DE 196 42 043 A werden Buprenorphin- oder Codein- enthaltende TTS für die Behandlung von Drogenabhängigkeit genannt. Das TTS enthält zwischen 5 und 20 % Wirkstoff auf Basis der Matrix, die ein Druckhaftkleber, basierend auf Polyacrylat und 5 bis 15 % einer öligen Vitamin E Lösung, sein kann.

In der WO 92 13540 A wird eine Salbe beschrieben, die ein oder mehrere Morphinderivate enthält. Diese Salbe wirkt als Analgetikum.

EP-A-0 672 416 offenbart eine stabile pharmazeutische Zusammensetzung, deren wesentlicher Bestandteil das Vorhandensein eines "hydrophobic, fusible carrier", d. h. einer hydrophoben, schmelzbaren Trägersubstanz ist, in die Diamorphin oder dessen Salz eingearbeitet ist. Hierunter ist ein natürliches oder synthetisches Wachs oder Öl zu verstehen, welches einen Schmelzpunkt im Bereich zwischen 35 und 150°C besitzt. Diese Zusammensetzung kann für die Behandlung von Drogensüchtigen eingesetzt werden.

In der deutschen Offenlegungsschrift DE 196 42 043 A1 ist noch die Verwendung von Acetylmethadol, Naltrexon, Codein, Dihydrocodein sowie Morphin zur Behandlung von Drogenabhängigkeit oder Drogensucht vorgeschlagen worden. Da bis auf Buprenorphin keiner der letztgenannten Wirkstoffe die analgetische Wirksamkeit von Heroin übertrifft, dürfte diesen Stoffen bei der Behandlung von Heroin-Schwerstabhängigen auch Grenzen gesetzt sein.

Obwohl es naheliegend scheint, den Wirkstoff Diamorphin als den Wirkstoff zu wählen, mit dem eine ausschleichende Therapie von Heroinabhängigkeit realisiert werden könnte, sind transdermale therapeutische Systeme zur kontinuierlichen und kontrollierten Freisetzung von Diamorphin zur Behandlung der Entzugsymtome bei Schwerstabhängigkeit durch Heroinsucht überraschenderweise noch nicht bekannt. Dies mag daran liegen, dass der Wirkstoff Diamorphin verhältnismässig leicht hydrolysiert und daher die Herstellung eines pharmazeutischen Präparats, welches diesen Wirkstoff über einen längeren Zeitraum abgeben soll und auch während der Zeit der vorangegangenen Lagerung keinen Zersetzungen unterliegen darf, bisher nicht gelungen ist.

Jedoch ist gerade eine solche Applikationsform für konstante Plasmaspiegel vorteilhaft, da durch den Weg über die Haut und geeignete Wahl der Grösse der Permeationsfläche ein therapeutisch sinnvoller Konzentrationskorridor in kontrollierter Weise erzielt wird. Konstante Plasmaspiegel ohne Spitzen, die für Nebenwirkungen verantwortlich sind und die mit schweren Entzugserscheinungen verbundene Unterversorgung sind jedoch die Voraussetzung für eine erfolgreiche Therapie unter dem Aspekt der Resozialisierung, wie ein seit 1994 in der Schweiz gross angelegter und wissenschaftlich ausgewerteter Feldversuch zur staatlich kontrollierten Heroinabgabe eindrucksvoll gezeigt hat (I. Weber, "Verschreibung von Heroin für Drogenabhängige", *Deutsche Apotheker Zeitung,* 138, 57, **1998**)

Aufgabe der vorliegenden Erfindung ist es, eine Vorrichtung und ein Verfahren zur Behandlung der genannten Symptome, die im Rahmen des abrupten Entzugs von Opiaten bei einem Süchtigen auftreten, zur Verfügung zu stellen, insbesondere für den speziellen Fall der Heroinsucht. Der Drogenabhängige (= Süchtiger = Patient) soll unter Vermeidung bzw. Reduzierung der Entzugssymptome der unkontrollierten Opiatzufuhr entzogen und schliesslich von einer regelmässigen Opiateinnahme befreit werden.

Eine weitere Aufgabe besteht darin, die Vorrichtung so zu konstruieren, dass der darin enthaltene aktive Wirkstoff über einen längeren Zeitraum abgegeben werden kann und zu gewährleisten, dass sich der aktive Wirkstoff während der Lagerung der Vorrichtung nicht zersetzt oder in einer anderen Weise seine Aktivität verliert.

Gelöst wird die Aufgabe durch eine Vorrichtung, die zur kontinuierlichen und kontrollierten Abgabe eines aktiven Wirkstoff befähigt ist, der vorzugsweise Diamorphinbase oder ein Säureadditionssalz von Diamorphin ist. Die Vorrichtung ist für eine Applikation auf der Haut oder der Schleimhaut geeignet, sowie für eine parenterale Applikation unter Einschaltung eines Resorptionsprozesses. Eine bevorzugte Vorrichtung ist ein transdermales therapeutisches System (TTS). Der Aufbau eines solchen Transdermalen Therapeutischen Systems ist dem Fachmann bekannt. Charakteristische Merkmale eines TTS sind mindestens eine wirkstoffenthaltende Schicht sowie ein Mittel zur Befestigung des TTS auf der Haut, im allgemeinen eine Haftkleberschicht. Die Vorrichtung kann aber auch eine oral applizierbare Darreichungsform sein, z. B. eine Tablette, eine Kapsel, oder ein Wafer.

Eine besondere Ausführungsform der Vorrichtung ist dadurch gekennzeichnet, dass der aktive Wirkstoff, z. B. Diamorphin und / oder ein pharmazeutisch verträgliches Säureadditionssalz von Diamorphin in einem pharmazeutisch verträglichen aprotischen Lösemittel und / oder einem pharmazeutisch verträglichen Lösemittel mit geringer protolytischer Aktivität enthalten ist wobei dieses Lösemittel mindestens zu 5% aus Vitamin E und / oder Vitamin E-Derivate besteht. Zu solchen pharmazeutisch verträglichen aprotischen Lösemitteln bzw. Lösemitteln mit geringer proteolytischer Aktivität zählen insbesondere N-Methylpyrrolidon, R-(+)-Limonen, Benzylnikotinat, Ölsäure, Dimethylisosorbid, Oleum citri und Tween 80.

Es hat sich gezeigt, dass solche Kombinationen von Vitamin E mit einem weiteren pharmazeutisch verträglichen aprotischen Lösemittel bzw. Lösemittel mit geringer proteolytischer Aktivität einen überraschend positiven stabilisierenden Effekt auf die Hydrolyse-Abbaurate von Diacetylmorphin zeigten. Die Ergebnisse dieser Untersuchungen sind Tabelle 1 zu entnehmen. Dieser Effekt ist insbeondere für die Langzeitstabilität der Vorrichtung von Bedeutung.

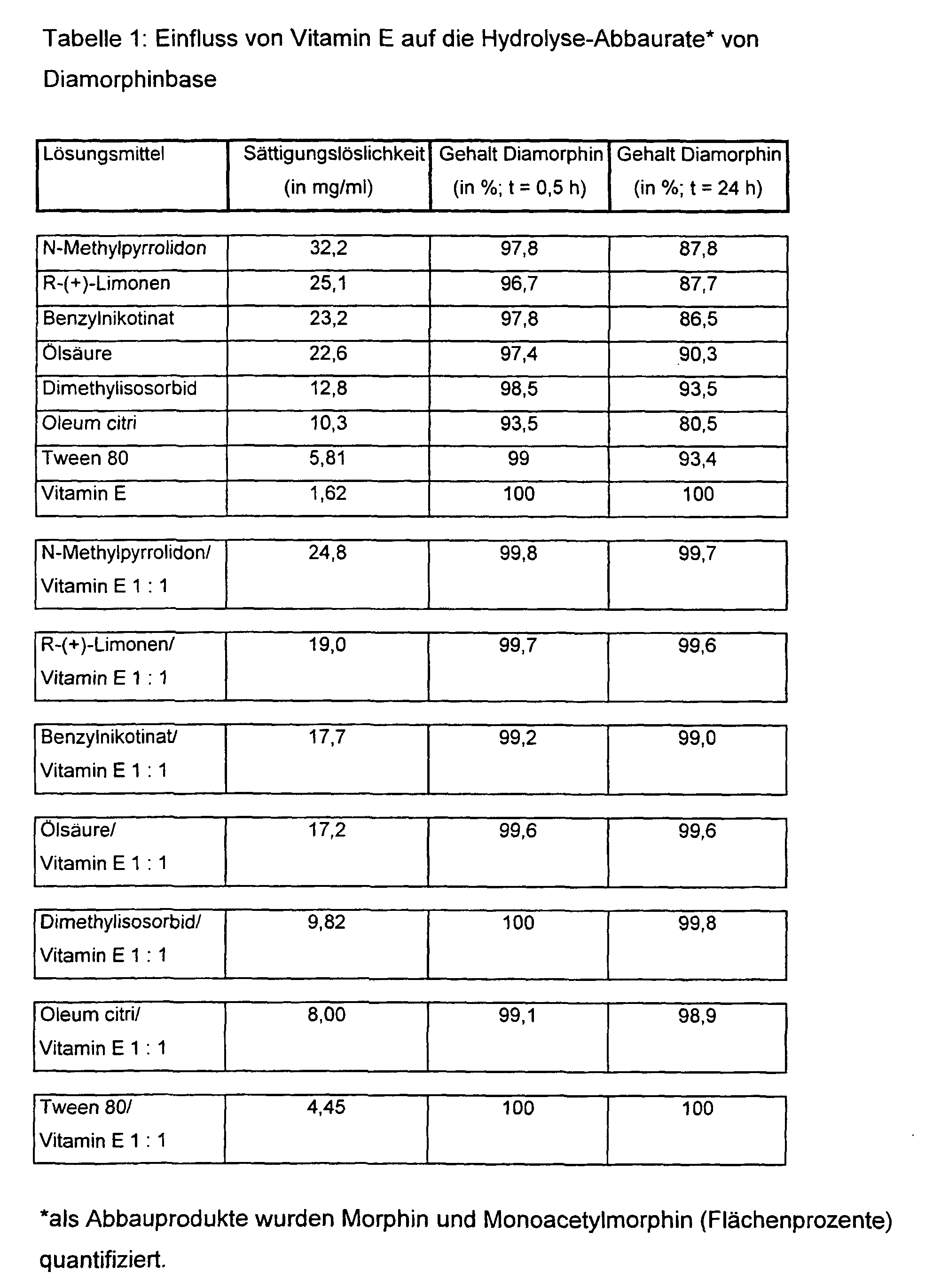

Es ist weiterhin vorteilhaft, gegebenenfalls während des Herstellungsverfahrens der Vorrichtung ein solches pharmazeutisch verträgliches aprotisches Lösemittel und / oder ein pharmazeutisch verträgliches Lösemittel mit geringer proteolytischer Aktivität als Lösemittel oder Trägersubstanz für den aktiven Wirkstoff zu verwenden.

In einer oralen Darreichungsform werden als aprotische Lösemittel bzw. als Lösemittel mit geringer proteolytischer Aktivität vorzugsweise solche verwendet, die einen Schmelzpunkt unterhalb von 35°C besitzen.

In einer weiteren bevorzugten Ausführungsform der Vorrichtung liegt der aktive Wirkstoff, insbesondere Diacetylmorphin, in einer pharmazeutischen Reinheit vor. Hierunter ist zu verstehen, dass ein Reinheitsgrad von 99% in Bezug auf die Gesamtmenge des aktiven Wirkstoffs erreicht wird. Die Gesamtmenge von nichtidentifizierbaren Fremdstoffen liegt unterhalb von 1%, besonders bevorzugt unterhalb von 0,1% bezogen auf den aktiven Wirkstoff.

Ein weiterer Vorteil der efindungsgemässen Vorrichtung ist die leichte Applizierbarkeit, die ein "seff-administering" ermöglicht. Dies ist für die Therapie nicht nur für den Patienten, sondern auch hinsichtlich einer ansonsten auftretendgen Zeitbelastung für Ärzte bzw. Pflegepersonal von grossem Vorteil.

Bei der Behandlung der Opiatsucht wird in einem ersten Phase dem Süchtigen (d. h. dem Patienten) der aktive Wirkstoff in kontinuierlicher und kontrollierter Weise zugeführt, wobei zunächst eine dem Tagesbedarf dieses Patienten angepasste Dosis verabreicht wird. Auf diese Weise erzielt man zunächst eine kontrollierte Opiatzufuhr, die die missbräuchliche und unkontrollierte Opiatzufuhr ersetzt.

In der zweiten Phase wird die Menge des dem Patienten kontinuierlich und kontrolliert zugeführten aktiven Wirkstoffs dann behutsam reduziert. Auf diese Weise erzielt man ein langsames und kontrolliertes Absinken des Morphinspiegels im Blut des Patienten (sog. "ausschleichende Therapie"). Zum Ende dieser zweiten Phase, welche sich ggf. in mehrere, d. h. mindestens zwei Stufen unterteilen lässt, kann im optimalen Fall auf eine weitere Zufuhr des aktiven Wirkstoffs gänzlich verzichtet werden.

Die beim unkontrollierten Entzug durch verringerte oder durch abrupt beendete Opiatzufuhr hervorgerufenen Entzugserscheinungen, also insbesondere die körperlichen und psychischen Entzugserscheinungen (z. B. die Entzugsschmerzen) werden auf diese Weise vermieden bzw. verringert. Ebenso wird die Intensität der bei missbräuchlicher Daueranwendung von Opiaten auftretenden Erscheinungen, nämlich der körperliche Abbau, psychische Störungen und intellektuelle Fehlleistungen vermindert.

Ein wichtiger Aspekt der Lösung ist somit auch eine sinnvolle Verabreichung des aktiven Wirksotffs, insbesondere von Diamorphin, die eine konstante Plasmakonzentration des aktiven Wirkstoffs bzw. des Hauptmetabolits Morphin innerhalb eines therapeutischen sinnvollen Konzentrationskorridors über einen definierten Zeitraum hinweg ermöglicht.

Die Morphine unterscheiden sich von den meisten Alkaloiden durch ihren komplizierteren Molekülbau. So kann man die Morphine selbst als Derivate des Isochinolins betrachten, andererseits kann man das Phenanthrenskelett als eigentliches Stammringsystem der Morphinalkaloide ansehen. Durch Variation der Grundstruktur des Morphins [(-)-Morphin = C₁₈H₂₁NO₃], d. h. durch gezielte Derivatisierung an bestimmten Stellen des Moleküls, lassen sich die unterschiedlichen Wirkmerkmale jeweils hervorheben. Bei dauerhafter Anwendung von Opiaten tritt bereits nach sehr kurzer Zeit ein Gewöhnungseffekt ein, so dass - um weiterhin eine Wirkung zu erzielen - die Dosis erhöht werden muss.

Auch Diamorphin leitet sich als semisynthetisches Opioid von Morphin ab; es zählt deshalb ebenfalls zur Gruppe der Opiate. Dargestellt wird es durch zweifache Acetylierung der phenolischen und alkoholischen Hydroxy-Gruppe des Morphins. Stoffe mit Acetylgruppen sind sehr hydrolyseanfällig. Diamorphin wird deshalb auch auf dem Wege der Biotransformation über Monoacetylmorphin wieder zu Morphin abgebaut; Morphin ist somit als Hauptmetabolit die eigentlich wirksame Form des Diamorphins. Der hydrolytische Abbau von Diamorphin zu Morphin vollzieht sich sogar in aprotischen Lösemitteln, wenn auch nicht in so dramatischer Weise wie in protischen Lösemitteln.

Diamorphin (Heroin, Diacetylmorphin) gehört also wie z. B. auch Hydromorphon und Buprenorphin in die Gruppe der partialsynthetisch hergestellten Morphinderivate. Es besitzt den grundsätzlichen Wirkcharakter der Opiate, zu dem an erster Stelle die Dämpfung reflektorischer Reaktionen des Organismus auf störende Einflüsse gehört. Somit wirkt die Substanzklasse stark schmerzstillend, hustenreizlindernd, anxiolytisch und antiemetisch. Die Morphine sind aber gleichzeitig auch opstipierend und hungerstillend.

### Dem besseren Verständnis der Erfindung dienen die folgenden Definitionen:

Zu den Patienten zählen Personen, die unter einer Opiatsucht leiden und somit von einer regelmässigen, im wesentlichen unkontrollierten Opiatzufuhr abhängig sind. Die Begriffe "Sucht", "Abhängigkeit" und "Missbrauch" usw. sollen im Sinne der vorliegenden Beschreibung synonym betrachtet werden, auch wenn in Fachkreisen diese Begriffe gelegentlich unterschiedlich definiert werden. Die Art des Drogenmissbrauchs, die durch die vorliegende Erfindung behandelt werden soll, ist jedoch die Drogenabhängigkeit vom Opiat-Typ. Der Opiatabhängige (z. B. Heroinabhängige) wird in dem Moment zum Patienten, sobald er sich entschliesst, sich einer Entzugstherapie zu unterziehen.

Bei den betreffenen suchtverursachenden Opiaten handelt es sich um solche, die bei missbräuchlicher Dauereinnahme abhängig machen, also Morphin-Alkaloide wie Heroin, Morphin, Opium oder Cocain, aber auch Kombinationen dieser Stoffe untereinander oder mit anderen Rauschmitteln bzw. Rauschgiften (wie Alkohol, Nikotin, Amphetamine, Cannabis, Barbiturate etc.).

Bei den Patienten, die sich einem Entzug unterziehen möchten, treten bei abrupter Unterbrechung der Opiatzufuhr Entzugserscheinungen auf, und zwar körperliche wie auch psychische. Hierzu zählen: ein als "craving" bezeichnetes besonderes Verlangen nach dem Opiat, Depression und depressive Stimmungen, Reizbarkeit, Antriebslosigkeit, Motivationsmangel, Appetitlosigkeit und veränderte Essgewohnheiten, Übelkeit, Zittern, Unruhe, psychomotorische Veränderungen und irreguläres Schlafverhalten.

Die Vorrichtung zur kontrollierten und kontinuierlichen Abgabe ist gekennzeichnet durch einen Gehalt eines aktiven Wirkstoffs. Bei dem aktiven Wirkstoff handelt es sich um Derivate des lsochinolins und / oder Derivate des Phenanthrens. Hiermit sind u. a. Morphinalkaloide gemeint, also Derivate des (-)-Morphins und / oder pharmazeutisch verträgliche Säureadditionssalze der Derivate von Morphin. Bevorzugter aktiver Wirkstoff ist Diamorphin, welcher als Diamorphinbase und / oder in Form eines pharmazeutisch verträglichen Säureadditionssalzes von Diamorphin vorliegt. Der aktive Wirkstoff kann aber auch die Kombination von mindestens einem Derivat des (-)-Morphin mit nicht-derivatisiertem, d. h. chemisch unverändertem (-)-Morphin sein. Der aktive Wirkstoff kann schliesslich auch in Form einer Einschlussverbindung, z. B. in Cyclodextrinen, und / oder an lonenaustauscherharze adsorbiert vorliegen.

Zu den pharmazeutisch verträglichen Säureadditionssalzen des aktiven Wirkstoffs zählen die Salze, die sich bei Reaktion der basischen Zentren der Morphin-Alkaloide mit entsprechenden Säuren bilden. Zu den geeigneten Säure zählen Salzsäure, Schwefelsäure, Bromwasserstoff, Milchsäure, Ameisensäure, Propionsäure, Essigsäure, Ölsäure, Phosphorsäure, Citronensäure, Ascorbinsäure und Weinsäure. Als pharmazeutisch verträgliche Säureadditionssalze bilden sich dabei Hydrochloride, Sulfate und Hydrogensulfate, Hydrobromide, Jodide, Lactate, Acetate, Formiate, Propionate, Oleate, Phosphate und Hydrogenphosphate, Citrate, Ascorbate und Tartrate. Bevorzugte pharmazeutisch verträgliche Säureadditionssalze von Diamorphin sind Diamorphin-Hydrochlorid, Diamorphin-Hydrogensulfat, Diamorphin-tartrat, Diamorphin-citrat, Diamorphin-acetat, Diamorphin-lactat und Diamorphin-Hydrobromid.

Die Vorrichtung kann die Form einer Lösung, einer Suspension, einer Emulsion, eines Schaumes, eines Implantats, einer Salbe, einer Paste, eines Suppositoriums, einer Tablette, eines Pulvers, eines Dragees, eines Sprays oder eines Aerosols besitzen. Die bevorzugte Form der Vorrichtung ist ein Transdermales Therapeutisches System.

Die Applikation der Vorrichtung erfolgt auf der Haut oder Schleimhaut des Patienten. Das heisst, dass die Applikationsarten transdermal, mucosal, bukkal, lingual, sublingual, enteral (= peroral), intestinal, nasal, rektal und per inhalationem sind. Sofern die Vorrichtung eine Lösung oder ein Implantat ist, kann auch eine parenterale Applikation in das Körperinnere des Patienten erfolgen. Diese Applikationsart erfolgt aber ausschliesslich unter Einschaltung eines Resorptionsprozesses, also intrakutan, subkutan, intramuskulär oder intraperitoneal.

Die Vorrichtung ist weiterhin befähigt, den aktiven Wirkstoff kontrolliert und kontinuierlich freizusetzen. Hierunter ist zu verstehen, dass der aktive Wirkstoff über einen längeren Zeitraum an den Applikationsort abgegeben wird. Als Applikationsort kommen die unverletzte Haut, Mund-, Zungen-, Nasen-, Magen-, Darm- und Rektumschleimhaut sowie die Bronchial- und Alveolarepithel in Frage. Im Fall der parenteralen Applikation unter Einschaltung eines Resorptionsprozesses sind die Haut, die Unterhaut, die Muskel und die Bauchhöhle geeignete Applikationsorte. In jedem Falle erfolgt die Freisetzung des aktiven Wirkstoffs auf kontrollierte, also in zeitlich verzögerter Weise.

Unter einem solchen längeren Zeitraum ist ein mindestens mehrstündiger Zeitraum zu verstehen. In Frage kommen Zeiträume von mindestens etwa 6, 12 oder 16 Stunden. Bevorzugt sind jedoch Zeiträume von etwa 24, 48 oder 72 Stunden. Im Fall eines Implantats kann sich der längere Zeitraum auch auf mindestens etwa 3 bis 7 Tage, bevozugt jedoch mindestens etwa 14 Tage bis etwa 3 Monate erstrecken. Im Fall, dass die Vorrichtung ein Transdermales Therapeutisches System ist, sind bevorzugte Zeiträume etwa 16, 24, 48 oder 72 Stunden.

Zu den vorzugsweise während der Herstellung der Vorrichtung als Lösemittel oder Träger für den aktiven Wirkstoff verwendeten pharmazeutisch verträglichen aprotischen Lösemitteln und / oder den pharmazeutisch verträglichen Lösemitteln mit geringer protolytischer Aktivität zählen N-Methylpyrrolidon, Nicotinsäurebenzylester, R-(+)-Limonen, Oleum citri, Ölsäure, Undecensäure, Dimethylisosorbid, Polyoxyethylen-Sorbitan-Monolaurat, Polyoxyethylen-Sorbitan-Monooleat, Polyoxyethylen-Sorbitan-Monopalmitat, Polyoxyethylen-Sorbitan-Monostearat, Polyoxyethylen-Sorbitan-Trioleat, Polyoxyethylen-Sorbitan-Tristearat (die auch in den unter dem Handelsnamen Tween 20, 40, 60, 80 oder 85 bekannten Produkten enthalten sind) und deren Kombinationen.

Als besonders bevorzugtes Lösemittel oder Träger für den aktiven Wirkstoff kommen Vitamin E, also (+)-α-Tocopherol, DL-α-Tocopherol und Derivate von Vitamin E wie Vitamin E-acetat und Vitamin E-succinat in Frage.

Die Behandlung der Opiatsucht, in dessen Rahmen die Vorrichtung verwendet wird, kann in zwei Stadien unterteilt werden. Die Dauer des Verfahrens hängt von der Schwere der Opiatsucht ab. Es ist klar, dass eine "Rund-um-die Uhr-Versorgung" mit dem aktiven Wirkstoff besonders vorteilhaft ist, da mitunter bereits wenige Stunden nach der letztmaliger Verabreichung des suchtverursachenden Opiats die besagten Entzugssymptome auftreten können.

In der ersten Phase wird dem Patienten der aktive Wirkstoff in kontinuierlicher und kontrollierter Weise zugeführt, wobei zunächst eine dem bisherigen Tagesbedarf dieses Patienten angepasste Dosis täglich verabreicht wird. Gleichzeitig wird auf eine missbräuchliche, d. h. unkontrollierte Opiatzufuhr vollständig verzichtet. Die Dauer dieser ersten Phase des Verfahrens kann mehrere Tage oder Wochen betragen.

In der zweiten Phase die sich ggf. in mehrere Stufen unterteilen lässt, wird die Menge des dem Patienten kontinuierlich und kontrolliert zugeführten aktiven Wirkstoffs behutsam reduziert. Hierunter ist zu verstehen, dass die täglich verabreichte Dosis leicht unter dem bisherigen Tagesbedarf des Patienten liegt. Eine solche Dosis wird in der ersten Stufe über einen Zeitraum von mehreren Tagen oder Wochen verabreicht. In weiteren Stufen wird wiederum jeweils eine Reduzierung der täglich verabreichten Dosis unter das Niveau der in der jeweils vorangegangenen Stufe täglich verabreichten Dosis vorgenommen. Auf diese Weise erfolgt eine stufenweise Reduzierung der täglich zugeführten Dosis des aktiven Wirkstoffs, wobei jede Stufe sich jeweils über einen Zeitraum von mehreren Tagen oder Wochen erstrecken kann. Ein exakter Therapieplan mit genauen Angaben der in diesen Stufen täglich zu verabreichenden Dosis kann jedoch nur vom Arzt individuell für jeden Patienten entsprechend der Schwere dessen Opiatabhängigkeit aufgestellt werden.

Auf diese Weise erzielt man ein langsames und kontrolliertes Absinken des Morphinspiegels im Blut des Patienten (sog. "ausschleichende Therapie"). Zum Ende dieser zweiten Phase kann auf eine weitere Zufuhr des aktiven Wirkstoffs gänzlich verzichtet werden. Die gesamteTherapie kann sich über mehrere Wochen oder Monate erstrecken.

Im Rahmen einer solchen Therapie wird eine Vorrichtung, die für eine einmal tägliche vorgesehen ist, d. h. die zu einer 24-stündigen kontinuierlichen und kontrollierten Abgabe des aktiven Wirkstoffs befähigt ist, einmal täglich durch eine entsprechende neue Vorrichtung ersetzt. Wird eine Vorrichtung verwendet, die für eine zwei- oder dreitägige Verabreichung vorgesehen ist, so muss diese Vorrichtung im Rahmen der Therapie entsprechend nur jeden zweiten bzw. dritten Tag durch eine neue Vorrichtung ausgetauscht werden.

Im Rahmen einer solchen Therapie wird eine erfindungsgemässe Vorrichtung angewendet, die den aktiven Wirkstoff kontrolliert und kontinuierlich freisetzt. Handelt es sich bei der einzusetzenden Vorrichtung um ein transdermales therapeutisches Systems mit dem aktiven Wirkstoff, welches diesen Wirkstoff über 16h lang kontrolliert abgibt, wird dieses z. B. morgens auf unverletzter Haut angebracht, tagsüber etwa 16 Stunden lang getragen und vor dem Zubettgehen abgezogen. Am nächsten Morgen wird dann das nächste 16-Stunden-TTS appliziert. Bei Verwendung eines transdermalen therapeutischen Systems mit dem aktiven Wirkstoff, welches diesen Wirkstoff über 24h lang kontrolliert abgibt, wird dieses z. B. morgens auf unverletzter Haut angebracht, tagsüber und in der kommenden Nacht getragen und am nächsten Morgen gegen das nächste 24-Stunden-TTS ausgetauscht. Entsprechend dem Ziel der stufenweise Reduzierung der täglich zugeführten Dosis des aktiven Wirkstoffs in der zweiten Phase des Verfahrens wird jeweils zu Beginn einer neuen Stufe eine erfindungsgemässe Vorrichtung verwendet, die eine geringere Menge des aktiven Wirkstoffs freisetzt.

Bei Verwendung einer erfindungsgemässen Vorrichtung, die den aktiven Wirkstoff über einen noch längeren Zeitraum kontinuierlich und kontrolliert abgibt, wird diese in entsprechenden Zeiträumen gegen neue Vorrichtungen ausgetauscht. In einer besonderen Ausführungsform kann der Beginn einer neuen Stufe der zweiten Phase, durch die eine gegenüber der in der vorangegangenen Stufe täglich verabreichten Dosis reduzierte tägliche Dosis verabreicht wird, mit der Verabreichung einer neuen Vorrichtung mit geringerer Wirkstofffreisetzung übereinstimmen. Dies kann z. B. bei Verwendung eines Implantats erfolgen.

Auf jeden Fall wird durch die erfindungsgemässen Vorrichtungen während der ersten Phase und während jeder Stufe der zweiten Phase ein konstanter Plasmaspiegel erzielt. Auf diese Weise ist gewährleistet, dass während der gesamten Dauer des Verfahrens, eine kontrollierte Morphinkonzentration im Blut des Patienten vorliegt.

Die Therapie, d. h. das erfindungsgemässe Verfahren, kann gegebenenfalls mindestens zeitweise unter Zusatz von kreislaufstützenden Medikamenten, Vitaminen, Tranquilizern, etc. durchgeführt werden. Es ist ebenfalls von Vorteil, die Therapie durch zusätzliche psychologische Betreuung zu unterstützen.

Im folgenden sind konkrete Ausführungsformen der erfindungsgemässen Vorrichtung beschrieben:

In einer oralen Formulierung ist der Wirkstoff in einer semipermeablen Membran eingekapselt, die z. B. Celluloseacetat enthält. Mit einem Bohrer oder Laser wird in das Kapselmaterial eine kleine Öffnung gebohrt. Im Körper des zu behandelnden Patienten wird nach Einnahme der Vorrichtung durch das Kapselmaterial hindurch Wasser absorbiert. Der aktive Wirkstoff wird dann durch osmotischen Druck in der gewünschten kontinuierlichen und kontrollierten Weise durch die kleine Öffnung nach aussen abgegeben. Solche Systeme sind beispielsweise in den US-Patenten 3,760,805 und US 3,987,790 beschrieben. In diesen Systemen können die pharmazeutischen Wirkstoffe in fester Form oder adsorbiert an lonenaustauscher-Harze vorliegen.

In einer (oral verabreichbaren) mucoadhäsiven Ausführungsform der Vorrichtung ist der aktive Wirkstoff in einer bioadhäsiven, biokompatiblen, wasserlöslichen und / oder zumindestens wasserquellfähigen Polymermatrix eingearbeitet. Eine solche Polymermatrix kann z. B. Polyacrylsäurecarboxymethylcellulose und weitere, aus EP 421 454 A bekannte "in Wasser quellbare Polymere" enthalten. Die mucoadhäsiven Vorrichtungen können im Aufbau denen von transdermalen Systemen ähnlich sein, ausser dass die Wirkstofffreisetzung auch zweiseitig, d. h. sowohl in Richtung Mucosa als auch in deren abgewandte Richtung (also in die Körperhöhle, Z. B: Magen, Darm etc.) erfolgen kann. Der Aufbau verschiedener mucoadhäsiver Systeme ist beschrieben bei Ahuja, Khar, Ali in *Drug Development and Industrial Pharmacy,* 23(5), 489-515 (**1997**).

Ein Transdermales Therapeutisches System ist eine mehrschichtig aufgebaute pharmazeutische Darreichungsform. Es enthält eine Rückschicht, die für den/die aktiven Wirkstoff(e) undurchlässig ist und eine haftklebende Schicht, die den/die aktiven Wirkstoff(e) enthält. Es gibt auch TTS-Typen, bei denen die Freisetzung des Wirkstoffs aus einem Reservoir durch eine semipermeable oder mikroporöse Membran gesteuert wird. Grundlegende TTS-Typen sind detailliert von Y. W. Chien in "Developmental Concepts and Practice in Transdermal Therapeutic Systems" in Y. W. Chien, *Transdermal Controlled Systemic Medications,* Marcel Dekker Inc., New York (**1982**), Chapter 2, p. 25-81 beschrieben. Um Wiederholungen zu vermeiden, soll hiermit der relevante Inhalt dieses Kapitels als Teil der Offenbarung dieser Erfindung aufgenommen werden.

Als TTS-Matrix der vorliegenden Erfindung kommen bevorzugt nichtwasserlösliche Haftkleber (engl.: pressure sensitive adhesives), z. B. auf Basis von Polyacrylat, Polymethacrylat, Polyisobutylen, Silikon, Styrol/Butadien-Copolymerisat oder Styrol/Isopren- Copolymerisat in Frage, wobei als Haftmatrix Polymere auf Acrylat- und/oder Methacrylatbasis, insbesondere Acrylatcopolymere aus 2-Ethylhexylacrylat, Vinylacetat, Acrylsäure und Glycidylmethacrylat mit bzw. ohne Titanchelatester, besonders bevorzugt sind.

Weitere Ausführungsformen geeigneter transdermaler therapeutischer Systeme sind in dem deutschen Patent DE 33 15 272 (entsprechend US 4,769,028) beschrieben. Dieses System besteht aus einer undurchlässigen Deckschicht, einem damit verbundenen, besonders aufgebauten übersättigten Wirkstoff-Reservoir aus einer Polymermatrix, einer mit dem Reservoir verbundenen, für den Wirkstoff durchlässigen Haftkleberschicht und einer die Haftkleberschicht abdeckenden, zum Gebrauch wieder ablösbaren Schutzschicht. Auch Systeme, in denen die Reservoirschicht eine so hohe Eigenklebrigkeit aufweist, dass sie gleichzeitig die Haftkleberschicht darstellt, sind möglich. Das deutsche Patent DE 38 43 239 (entsprechend US 5,089,267) beschreibt ein solches System.

Andere geeignete transdermale Vorrichtungen sind in den Patenten US 3,742,951, US 3,797,494, US 3,996,934 und US 4,031,894 beschrieben. Diese TTS-Typen bestehen grundsätzlich aus einer Rückschicht, die eine der Oberflächen darstellt, einer für den Wirkstoff durchlässigen Klebschicht, die die andere Oberfläche darstellt und letztlich einem Reservoir, das den Wirkstoff zwischen den beiden die Oberfläche bildenden Schichten enthält. Alternativ dazu kann der Wirkstoff auch in einer Vielzahl von Mikrokapseln enthalten sein, die in der durchlässigen Klebschicht verteilt sind. In jedem Fall wird der Wirkstoff aus dem Reservoir oder den Mikrokapseln durch eine Membran in die für den Wirkstoff durchlässige Klebschicht, die im Kontakt mit der Haut oder Schleimhaut des Patienten steht, kontinuierlich abgegeben. Im Falle von Mikrokapseln kann das Kapselmaterial auch als Membran wirken.

Die Konzentration des aktiven Wirkstoffs hängt von der Grösse der wirkstofffreisetzenden Fläche des Reservoirs ab. So kann der Gehalt von aktivem Wirkstoff zwischen 0,1 bis 50 Gew.-% der Polymermatrix betragen. Besonders bevorzugt ist ein Gehalt zwischen 10 bis 15 Gew.-% an aktivem Wirkstoff.

Bevorzugte Transdermale Therapeutische Systeme besitzen eine den aktiven Wirkstoff mit einem Gehalt von 3 bis 25 Gew.-% enthaltende Schicht. Besonders bevorzugt ist ein Gehalt von 10 bis 20 Gew.-%, z. B. Diamorphin und / oder ein pharmazeutisch verträgliches Säureadditionssalz von Diamorphin.

Die erfindungsgemässen Transdermalen Therapeutischen Systeme können in der den aktiven Wirkstoff enthaltenden Schicht zusätzlich mindestens ein Lösemittel oder Träger für den aktiven Wirkstoff enthalten.

Die Matrix des erfindungsgemässen TTS kann weiterhin Vitamin E oder ein Vitamin E-Derivat enthalten. In einer bevorzugten Ausführungsform liegt dieser Gehalt zwischen 5 - 15 Gew-%, bezogen auf das Gesamtgewicht der Polymermatrix. In einer weiteren bevorzugten erfindungsgemässen Ausführungsvariante ist Vitamin E als ölige D-α-Tocopherol-Lösung zu 6,25 Gew-% in der Polymermatrix enthalten. Der Zusatz von Vitamin E oder den genannte Derivaten verringert ausserdem die Löslichkeit des aktiven Wirkstoffs im Matrixmaterial. Hierdurch wird wiederum ein grösserer Hautflux begünstigt, da die thermodynamische Aktivität des aktiven Wirkstoffs in der Polymermatrix erhöht wird.

Die Abgabefläche der Wirkstoffmatrix ist durch den festen Verbund mit einer Trägerfolie ("backing layer") variierbar. Diese Folie, die in einer bevorzugten Ausführungsform wasserdampfundurchlässig ist, kann z. B. aus Polyester, Polypropylen oder beschichtetem Papier mit einer Schichtdicke von etwa 10 - 100 µm bestehen. Bei einer bevorzugten Ausführungsform besteht die Trägerschicht aus Polyethylenterephtalat (PET) mit einer Schichtdicke von 15 bis 50 µm, wobei das PET klar, opak oder bedruckt vorliegen kann.

Optional kann das TTS noch mit einem sogenannten Überpflaster oder "Coverpatch" aus textilem Gewebe ausgestattet sein, um eine zusätzliche Fixierung auf der Haut, z. B. bei starkem Schwitzen, zu erreichen.

Weiterhin ist das TTS durch eine wiederablösbare Schutzschicht ("release liner") mit einer Schichtdicke von etwa 40 bis etwa 100 µm charakterisiert. Die ablösbare Schutzschicht, die mit der Reservoirschicht in Berührung steht und vor Gebrauch vom TTS abgezogen werden muss, besteht beispielsweise aus denselben Materialien, wie sie zur Herstellung der Rückschicht benutzt werden. Die Wiederablösbarkeit wird erreicht z. B. durch Silikonbehandlung der Folienoberflächen. Optional kann die Schutzschicht neben der Silikonisierung zusätzlich noch metallisiert sein, z. B. durch Aluminiumbedampfung. Die Schutzschicht kann zusätzlich mit Applikationshilfen versehen sein, mit deren Hilfe sie leichter vom TTS abgezogen werden kann. Die einfachste Applikationshilfe ist ein Überstehen des Schutzschichtformates gegenüber dem wirkstoffhaltigen Matrixformat. Eine andere denkbare Applikationshilfe ist das Durchstanzen von verschiedenen Flächensegmenten der Schutzfolie. Bei einer bevorzugten Ausführungsform besteht die Schutzschicht aus Polyethylenterephtalat (PET) mit einer Schichtdicke von etwa 100 µm, wobei das PET wieder klar, opak oder bedruckt vorliegen kann.

Ein Vorteil der Vorrichtung sind die besonders niedrigen Kosten der Therapie. Dies liegt zum einen daran, dass es nicht erforderlich ist, dass fachkundiges Personal (Ärzte, Krankenschwestern) das Applizieren der Vorrichtung vornehmen muss. Ausserdem sind die beschriebenen Vorrichtungen kostengünstiger herzustellen als vergleichbare Vorrichtungen für eine parenterale Verabreichung unter Umgehung der Resorption, also etwa Infusionsflaschen und Injektionsspritzen.

Ein weiterer Vorteil der beschriebenen Vorrichtungen ist der Unterschied zu dem bei Missbrauch üblichen Injizieren oder Rauchen von Heroin. Zum einen bleiben Folgen von dauernden Injektionen, wie z. B. Abszesse oder sonstige Venenerkrankungen bzw. Emboliegefahr aus, zum anderen sind Infektionen wie HIV bzw. Hepatitis, die bei unsachgemässer Injektion auftreten können, ausgeschlossen.

Für diese Applikationsform des aktiven Wirkstoffs, insbesondere im Fall von Diamorphin in einem TTS spricht zusätzlich der Sicherheitsaspekt bezüglich eventuellen Missbrauchs. Eine missbräuchliche Verwendung der Vorrichtung ist im Gegensatz zu den bisher angewandten Darreichungsformen von Diamorphin nahezu ausgeschlossen, da eine Extraktion und Isolierung des Wirkstoffes aus einer TTS-Matrix ohne fachmännische Kenntnisse und aufwendigste Laborausstattung nicht möglich ist.

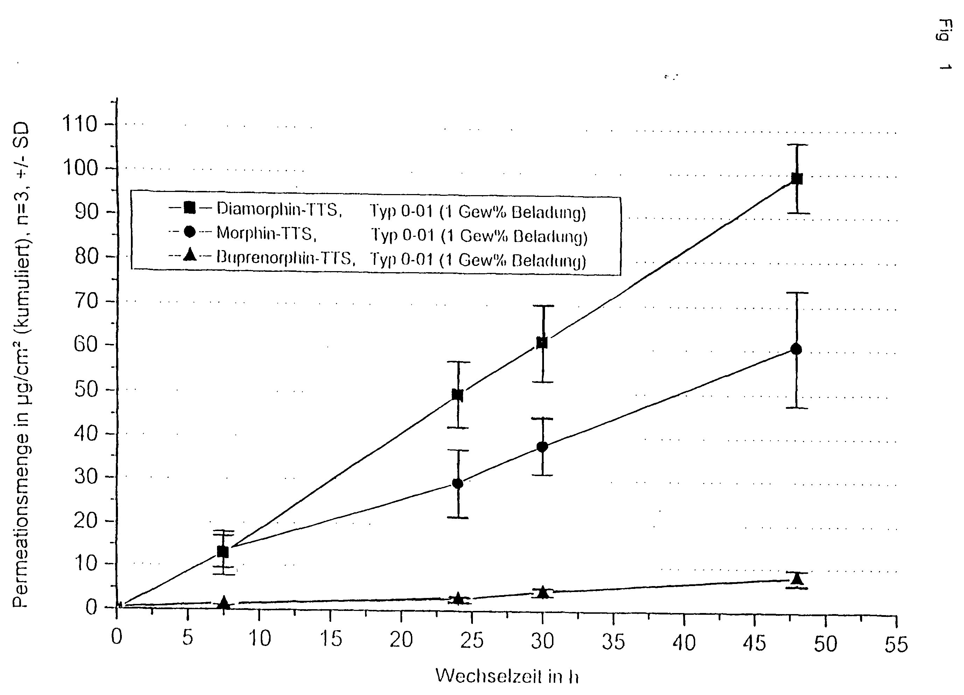

In Figur 1 wird die Hautpermeation (nude guinea pig) verschiedener Opiatbasen aus einem TTS gleicher Rezeptur (freigesetzt in Citratpuffer pH=3,0 +0,1 % NaN3 bei T=37 °C) verglichen. Die Bestimmung von Diamorphin erfolgte als Summe von Heroin + Metabolit Morphin.

Die Figur zeigt die gegenüber Morphin und Buprenorphin bessere Hautgängigkeit des Diamorphins, kontrolliert freigesetzt am in vitro-Hautmodell "nude guinea pig" aus einem Matrix-System gleicher Grundrezeptur und Beladung. Der Hautflux im Fliessgleichgewicht (Permeationsrate in µg/cm² x h) erstreckt sich mindestens bis zum Zeitpunkt t = 48 h und liegt auch teilweise deutlich höher als der von Morphin und Buprenorphin. Damit ist eine hervoragende kontinuierliche und kontrollierte transdermale Applikation des aktiven Wirkstoffs im Sinne der vorliegenden Erfindung zur Behandlung bei Heroinsucht belegt.

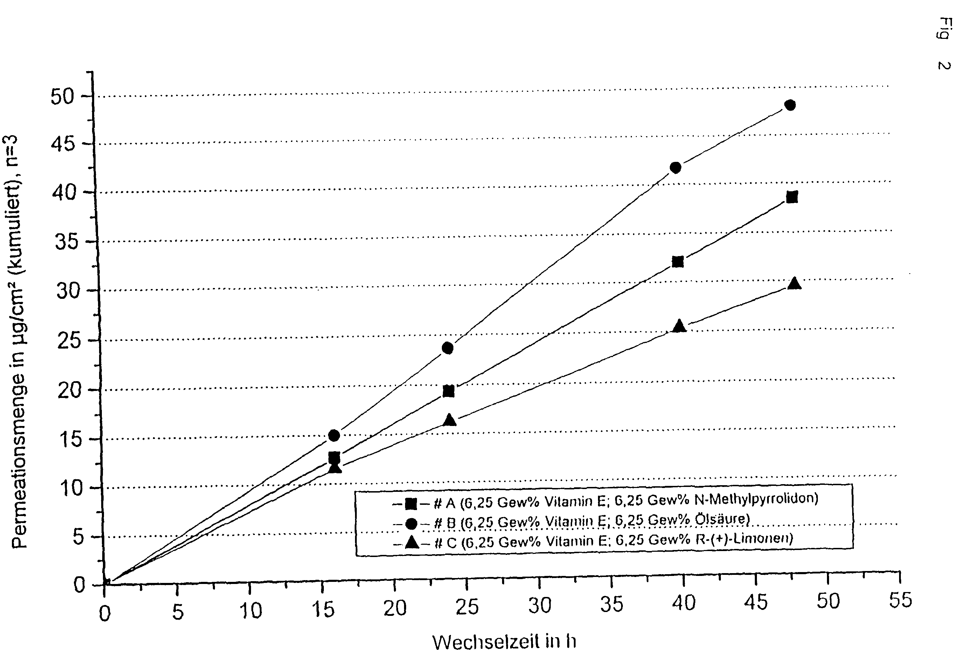

In Figur 2 wird die Hautpermeation (Human-Vollhaut, ID 446) verschiedener TTS von Diamorphinbase auf Polyacrylbasis (Wirkstoffgehalt=10,0 Gew.-%) verglichen.

Die Figur zeigt das Permeationsverhalten erfindungsgemässer TTS-Formulierungen unter in vitro-Bedingungen bei 37°C am Hautdiffusionsmodell "Humanhaut". Dazu wurden TTS mit einer Permeationsfläche von 1,54 cm² hergestellt, auf die Hautoberfläche von präparierten Human-Vollhautproben (kosmetisches Operationsmaterial von weiblichen Brustreduktionen ohne Unterhautfettgewebe) aufgeklebt und in modifizierten FRANZ-Diffusionszellen bezüglich ihrer Permeationsraten (Abhängigkeit der Konzentration von permeiertem Wirkstoff als Zeitfunktion im Akzeptormedium) mittels HPLC untersucht. Als Akzeptor diente 0,9%ige Kochsalzlösung mit 0,1 % Zusatz Natriumazid. Die TTS waren von gleicher Grundrezeptur und Wirkstoffbeladung. Sie unterschieden sich lediglich in der Auswahl des Lösungsmittels bzw. Weichmachers. Da der Hautflux (Permeationsrate in µg/cm² x h) im steady state (Fliessgleichgewicht) sich mindestens bis zum Zeitpunkt t = 48 h erstreckt, ist die Voraussetzung für eine gesteuerte transdermale Applikation über die Haut im Sinne der Behandlung bei Heroinsucht gegeben.

Der Erläuterung der Herstellung erfindungsgemässer Vorrichtungen dienen die folgenden Beispiele:

### Beispiel 1: Herstellung und die Rezepturbestandteile eines erfindungsgemässen Transdermalen Therapeutischen Systems.

0,3125 g D-α-Tocopherol (entspricht 6,25 Gew%) wurden in ein Rührgefäss vorgelegt und durch Hinzufügen von 0,3125 g N-Methylpyrrolidon (entspricht 6,25 Gew-%) in Lösung gebracht. In diese Lösung wiederum wurden unter Rühren 0,5 g Diamorphinbase (entspricht 10 Gew-%) portionsweise eingebracht. Unter Zusatz von 1 ml Ethylacetat wurde bis zum vollständigen Auflösen des Feststoffes (ca. 15 min, visuelle Kontrolle) gerührt. Diese Lösung wurde dann unter Rühren portionsweise in 10,39 g eines selbstvernetzenden Acrylatcopolymeren aus 2-Ethylhexylacrylat, Vinylacetat, Acrylsäure und Glycidylmethacrylat (37,3 Gew-% in einem Lösungsmittelgemisch aus Ethylacetat : 2-Propanol : Hexan von 54 : 35 : 11; Typ DuroTak 1753 der Fa. National Starch, Neustadt/Weinstrasse) eingerührt.

Anschliessend wurde der Masseansatz ca. 30 min bei Raumtemperatur gerührt und 15 min bei T = 40°C im Ultraschallbad nachbehandelt, um überschüssige Luft aus der Masse zu entfernen. Die Kleberlösung wurde dann mit einem geeigneten Rakel in einer Nassschichtdicke von 300 µm auf eine silikonisierte Polyethylenterephtalatfolie (Hostaphan RN 100 54B/54B der Fa. Hoechst, Frankfurt) ausgestrichen. Nachdem die Lösemittel durch 30minütiges Trocknen bei 50°C entfernt worden waren, wurde der Kleberfilm mit einer 15 µm dicken Polyesterfolie durch Kaschieren abgedeckt. Mit geeigneten Schneidewerkzeugen wurden die vorgesehenen Applikationsflächen ausgestanzt sowie die Ränder durch Abgittern entfernt.

### Beispiel 2: Herstellung und Rezepturbestandteile eines weiteren erfindungsgemässen Transdermalen Therapeutischen Systems.

Beispiel 1 wurde wiederholt, mit der Ausnahme, dass statt N-Methylpyrrolidon gleiche Gewichtsteile Ölsäure eingesetzt wurden.

### Beispiel 3: Herstellung und Rezepturbestandteile eines weiteren erfindungsgemässen Transdermalen Therapeutischen Systems.

Beispiel 1 wurde wiederholt, mit der Ausnahme, dass statt N-Methylpyrrolidon gleiche Gewichtsteile R-(+)-Limonen eingesetzt wurden.

## Patentansprüche

1. Pharmazeutische Vorrichtung zur kontinuierlichen und kontrollierten Abgabe mindestens eines aktiven Wirkstoffs für die Applikation auf der unverletzten Haut, auf der Mund-, Zungen-, Nasen- oder Rektumschleimhaut, auf der Bronchial- oder Alveolarepithel, oder parenteral unter Einschaltung eines Resorptionsprozesses, **dadurch gekennzeichnet, dass** der aktive Wirkstoff
a) Diamorphin ist, welcher als Diamorphinbase, in Form eines pharmazeutisch verträglichen Säureadditionssalzes aus der Gruppe bestehend aus dem Hydrochlorid, dem Sulfat, dem Hydrogensulfat, dem Hydrobromid, dem Jodid, dem Lactat, dem Acetat, dem Formiat, dem Propionat, dem Oleat, dem Phosphat, dem Hydrogenphosphat, dem Citrat, dem Ascorbat und dem Tartrat von Diamorphin oder in Form einer Einschlussverbindung vorliegt und
b) in einem pharmazeutisch verträglichen aprotischen Lösemittel und / oder einem pharmazeutisch verträglichen Lösemittel mit geringer protolytischer Aktivität enthalten ist, wobei das Lösemittel mindestens zu 5 Gew.-% aus Vitamin E und/oder Vitamin-E-Derivaten besteht.

2. Pharmazeutische Vorrichtung nach Anspruch 1, **dadurch gekennzeichnet, dass** sie die Form einer Lösung, einer Suspension, einer Emulsion, eines Implantats, eines Suppositoriums, einer Tablette, eines Pulvers, eines Dragees, eines Transdermalen Therapeutischen Systems, eines Wafers, eines Sprays oder eines Aerosols besitzt.

3. Pharmazeutische Vorrichtung nach einem der Ansprüche 1 bis 2, **dadurch gekennzeichnet, dass** der aktive Wirkstoff zusätzlich (-)-Morphin und / oder ein pharmazeutisch verträgliches Säureadditionssalz von (-)-Morphin enthält.

4. Pharmazeutische Vorrichtung nach einem der Ansprüche 1 bis 3, **dadurch gekennzeichnet, dass** sie die Form eines schichtförmig aufgebauten Transdermalen Therapeutischen Systems mit einer einen Haftkleber enthaltenden Schicht besitzt.

5. Pharmazeutische Vorrichtung nach Anspruch 4, **dadurch gekennzeichnet, dass** der Haftkleber Polymere enthält, die aus der Gruppe der Polymere auf Acrylat- und / oder Methacrylatbasis, Silikone, Polyisobutylene, Styroi/Butadien-Copolymerisate, Styrol/Isopren-Copolymerisate und den Estern des hydrierten Kolophoniums ausgewählt sind.

6. Pharmazeutische Vorrichtung nach Anspruch 4 oder 5, **dadurch gekennzeichnet, dass** die im Haftkleber enthaltenen Polymere auf Acrylat- und / oder Methacrylatbasis selbstvernetzende Acrylatpolymere sind.

7. Pharmazeutische Vorrichtung nach einem der Ansprüche 4 bis 6, **dadurch gekennzeichnet, dass** 2-Ethylhexylacrylat, Vinylacetat und Acrylsäure in der Monomerenzusammensetzung des selbstvernetzenden Acrylatpolymeren enthalten sind.

8. Pharmazeutische Vorrichtung nach einem der Ansprüche 4 bis 7, **dadurch gekennzeichnet, dass** eine Schicht 0,1 bis 50 Gew.-% des aktiven Wirkstoffs und / oder eines pharmazeutisch verträglichen Säureadditionssalzes enthält.

9. Pharmazeutische Vorrichtung nach einem der Ansprüche 4 bis 8, **dadurch gekennzeichnet, dass** das pharmazeutisch verträgliche aprotische Lösemittel und / oder das pharmazeutisch verträgliche Lösemittel mit geringer protolytischer Aktivität aus der Gruppe N-Methylpyrrolidon, Nicotinsäurebenzylester, R-(+)-Limonen, Oleum citri, Ölsäure, Undecensäure, Dimethylisosorbid, Polyoxyethylen-Sorbitan-Monolaurat, Polyoxyethylen-Sorbitan-Monooleat, Polyoxyethylen-Sorbitan-Monopalmitat, Polyoxyethylen-Sorbitan-Monostearat, Polyoxyethylen-Sorbitan-Trioleat, Polyoxyethylen-Sorbitan-Tristearat, Vitamin E, Derivate von Vitamin E und deren Kombinationen ausgewählt ist.

10. Pharmazeutische Vorrichtung nach einem der Ansprüche 4 bis 9, **dadurch gekennzeichnet, dass** die den aktiven Wirkstoff enthaltende Schicht 1 bis 15 Gew.-% Vitamin E und / oder ein Vitamin E-Derivat enthält.

11. Verfahren zur Herstellung einer pharmazeutischen Vorrichtung enthaltend als aktiven Wirkstoff Diamorphin, **dadurch gekennzeichnet, dass** während des Herstellungsverfahrens der Vorrichtung ein pharmazeutisch verträgliches aprotisches Lösemittel und / oder ein pharmazeutisch verträgliches Lösemittel mit geringer protolytischer Aktivität als Lösemittel oder Trägersubstanz für den aktiven Wirkstoff verwendet wird, wobei das Lösemittel mindestens 5 Gew.-% Vitamin E und / oder ein Vitamin E-Derivat enthält.

12. Verfahren zur Herstellung einer pharmazeutischen Vorrichtung nach Anspruch 4, **gekennzeichnet durch** die Schritte:
a) Einbringen des aktiven Wirkstoffs in die Lösung oder Schmelze einer polymeren Trägersubstanz,
b) Ausstreichen dieser wirkstoffhaltigen Polymerlösung oder Polymerschmelze auf eine Trägerbahn,
c) Verfestigung der ausgestrichenen wirkstoffhaltigen Polymerlösung oder Polymerschmelze **durch** Entfernung des Lösungsmittels oder der Lösungsmittel, **durch** Abkühlung oder **durch** Stehenlassen, ggf. unter Vernetzung des Polymers, und
d) Ausstanzen von Einzelpflastern aus der nach der Verfestigung erhaltenen wirkstoffhaltigen Polymermasse.

13. Verwendung eines Derivats des (-)-Morphins und / oder eines pharmazeutisch verträgliches Säureadditionssalz eines Derivats des (-)-Morphins zur Herstellung eines Arzneimittels zur Verwendung bei der kontinuierlichen und kontrollierten Verabreichung des aktiven Wirkstoffs bei einem Verfahren zur Behandlung von mit der bei abrupter Einstellung von Opiatmissbrauch verbundenen körperlichen und / oder psychischen Entzugssymptomen.

14. Verwendung nach Anspruch 13, **dadurch gekennzeichnet, dass** es sich bei dem Derivat des (-)-Morphins um Diamorphin handelt.

## Claims

1. Pharmaceutical device for continuous and controlled release of at least one active substance for application to the undamaged skin, to the oral, lingual, nasal or rectal mucosae, to the bronchial or alveolar epithelium, or parenterally with inclusion of an absorption process, wherein the active substance
a) is diamorphine, which is present as diamorphine base, in the form of a pharmaceutically compatible acid addition salt from the group consisting of the hydrochloride, the sulphate, the hydrogensulphate, the hydrobromide, the iodide, the lactate, the acetate, the formate, the propionate, the oleate, the phosphate, the hydrogenphosphate, the citrate, the ascorbate and the tartrate of diamorphine or in the form of an inclusion compound, and
b) is present in a pharmaceutically compatible aprotic solvent and/or a pharmaceutically compatible solvent having low proteolytic activity, the solvent consisting of at least 5 % by weight of vitamin E and/or vitamin E derivatives.

2. Pharmaceutical device according to Claim 1, **characterized in that** it possesses the form of a solution, suspension, emulsion, implant, suppository, plain tablet, powder, coated tablet, transdermal therapeutic system, wafer, spray, or aerosol.

3. Pharmaceutical device according to any of Claims 1 to 2, **characterized in that** the active substance further comprises (-)-morphine and/or a pharmaceutically compatible acid addition salt of (-)-morphine.

4. Pharmaceutical device according to any of Claims 1 to 3, **characterized in that** it possesses the form of a multilayer-constructed transdermal therapeutic system having a layer comprising a pressure-sensitive adhesive.

5. Pharmaceutical device according to Claim 4, **characterized in that** the pressure-sensitive adhesive comprises polymers selected from the group consisting of polymers based on acrylate and/or methacrylate, silicones, polyisobutylenes, styrene/butadiene copolymers, styrene/isoprene copolymers, and the esters of hydrogenated rosin.

6. Pharmaceutical device according to Claim 4 or 5, **characterized in that** the polymers present in the pressure-sensitive adhesive are self-crosslinking acrylate polymers based on acrylate and/or methacrylate.

7. Pharmaceutical device according to any of Claims 4 to 6, **characterized in that** it comprises 2-ethylhexyl acrylate, vinyl acetate and acrylic acid in the monomer composition of the self-crosslinking acrylate polymer.

8. Pharmaceutical device according to any of Claims 4 to 7, **characterized in that** one layer comprises from 0.1 to 50 % by weight of the active substance and/or of a pharmaceutically compatible acid addition salt.

9. Pharmaceutical device according to any of Claims 4 to 8, **characterized in that** the pharmaceutically compatible aprotic solvent and/or the pharmaceutically compatible solvent having low proteolytic activity is selected from the group consisting of N-methylpyrrolidone, benzyl nicotinate, R-(+)-limonene, lemon oil, oleic acid, undecenoic acid, dimethylisosorbide, polyoxyethylene sorbitan monolaurate, polyoxyethylene sorbitan monooleate, polyoxyethylene sorbitan monopalmitate, polyoxyethylene sorbitan monostearate, polyoxyethylene sorbitan trioleate, polyoxyethylene sorbitan tristearate, vitamin E, derivatives of vitamin E and combinations thereof.

10. Pharmaceutical device according to any of Claims 4 to 9, **characterized in that** the layer comprising the active substance comprises from 1 to 15 % by weight of vitamin E and/or a vitamin E derivative.

11. Process for producing a pharmaceutical device comprising as active substance diamorphine, **characterized in that** a pharmaceutically compatible aprotic solvent and/or a pharmaceutically compatible solvent having low proteolytic activity is used as a solvent or vehicle for the active substance while the device is being produced, the solvent comprising at least 5 % by weight of vitamin E and/or a vitamin E derivative.

12. Process for producing a pharmaceutical device according to Claim 4, **characterized by** the steps of:
a) introducing the active substance into the solution or melt of a polymeric vehicle,
b) coating this polymer solution or polymer melt, containing active substance, onto a carrier web,
c) solidifying the coated polymer solution or polymer melt, containing active substance, by removing the solvent or solvents, by cooling or by leaving it to stand, with or without crosslinking of the polymer, and
d) punching individual patches from the polymer composition, containing active substance, that is obtained after the solidifying.

13. Use of a derivative of (-)-morphine and/or of a pharmaceutically compatible acid addition salt of a derivative of (-)-morphine for producing a medicament for use in the continuous and controlled administration of the active substance in a process for treating psychological and/or physical withdrawal symptoms associated with the sudden termination of opiate misuse.

14. Use according to Claim 13, **characterized in that** the derivative of (-)-morphine is diamorphine.

## Revendications

1. Dispositif pharmaceutique pour la délivrance continue et contrôlée d'au moins un principe actif pour l'administration sur la peau non lésée, sur la muqueuse buccale, linguale, nasale ou rectale, sur l'épithélium bronchique ou alvéolaire, ou par voie parentérale par l'insertion d'un processus de résorption, **caractérisé en ce que** le principe actif
a) est de la diacétylmorphine, laquelle se trouve comme diacétylmorphine base, sous forme d'un sel d'addition d'acide pharmaceutiquement acceptable appartenant au groupe constitué du chlorhydrate, du sulfate, de l'hydrogénosulfate, de l'hydrobromure, de l'iodure, du lactate, de l'acétate, du formiate, du propionate, de l'oléate, du phosphate, de l'hydrogénophosphate, du citrate, de l'ascorbate et du tartrate de diacétylmorphine ou sous forme d'un composé d'inclusion et
b) est inclus dans un solvant aprotique pharmaceutiquement acceptable et/ou dans un solvant pharmaceutiquement acceptable présentant une faible activité protolytique, sachant que le solvant se compose d'au moins 5 % en poids de vitamine E et/ou de dérivés de vitamine E.

2. Dispositif pharmaceutique selon la revendication 1, **caractérisé en ce qu'**il a la forme d'une solution, d'une suspension, d'une émulsion, d'un implant, d'un suppositoire, d'un comprimé, d'une poudre, d'une dragée, d'un système thérapeutique transdermique, d'une plaquette, d'un spray ou d'un aérosol.

3. Dispositif pharmaceutique selon l'une quelconque des revendications 1 et 2, **caractérisé en ce que** le principe actif contient en outre de la (-)-morphine et/ou un sel d'addition d'acide pharmaceutiquement acceptable de (-)-morphine.

4. Dispositif pharmaceutique selon l'une quelconque des revendications 1 à 3, **caractérisé en ce qu'**il a la forme d'un système thérapeutique transdermique disposé en couches avec une couche contenant un adhésif de contact.

5. Dispositif pharmaceutique selon la revendication 4, **caractérisé en ce que** l'adhésif de contact contient des polymères qui sont choisis dans le groupe de polymères composé de l'acrylate base et/ou du méthacrylate base, du silicone, du polyisobutylène, des copolymères styrol/butadiène, des copolymères styrol/isoprène et des esters de colophane hydraté.

6. Dispositif pharmaceutique selon la revendication 4 ou 5, **caractérisé en ce que** les polymères contenus dans l'adhésif de contact sont des polymères d'acrylate autoréticulants d'acrylate base et/ou de méthacrylate base.

7. Dispositif pharmaceutique selon l'une quelconque des revendications 4 à 6, **caractérisé en ce que** le 2-éthylhexylacrylate, le vinylacétate et l'acide acrylique sont contenus dans la composition des monomères des polymères d'acrylate autoréticulants.

8. Dispositif pharmaceutique selon l'une quelconque des revendications 4 à 7, **caractérisé en ce qu'**une couche contient 0,1 à 50 % en poids de principe actif et/ou un sel d'addition d'acide pharmaceutiquement acceptable.

9. Dispositif pharmaceutique selon l'une quelconque des revendications 4 à 8, **caractérisé en ce que** le solvant aprotique pharmaceutiquement acceptable et/ou le solvant pharmaceutiquement acceptable présentant une faible activité protolytique est choisi parmi le groupe composé de la N-méthylpyrrolidone, le benzylester d'acide nicotinique, les R-(+)-limonènes, l'essence de citron, l'acide oléique, l'acide undécylique, le diméthyl isosorbide, le polyoxyéthylène sorbitan monolaurate, le polyoxyéthylène sorbitan mono-oléate, le polyoxyéthylène sorbitan monopalmitate, le polyoxyéthylène sorbitan monostéarate, le polyoxyéthylène sorbitan trioléate, le polyoxyéthylène sorbitan tristéarate, la vitamine E, des dérivés de la vitamine E et leurs combinaisons.

10. Dispositif pharmaceutique selon l'une quelconque des revendications 4 à 9, **caractérisé en ce que** la couche contenant le principe actif contient 1 à 15 % en poids de vitamine E et/ou de dérivé de vitamine E.

11. Procédé pour la fabrication d'un dispositif pharmaceutique contenant de la diacétylmorphine comme principe actif, **caractérisé en ce que**, pendant le processus de fabrication du dispositif, un solvant aprotique pharmaceutiquement acceptable et/ou un solvant pharmaceutiquement acceptable présentant une faible activité protolytique est utilisé comme solvant ou substance vectrice du principe actif, sachant que le solvant contient au moins 5 % en poids de vitamine E et/ou d'un dérivé de vitamine E.

12. Procédé pour la fabrication d'un dispositif pharmaceutique selon la revendication 4, **caractérisé par** les étapes suivantes :
a) introduction du principe actif dans la solution ou la masse fondue d'une substance vectrice polymérique,
b) étalement de cette solution polymérique ou de cette masse fondue polymérique contenant le principe actif sur une bande vectrice,
c) solidification de la solution polymérique ou de la substance fondue polymérique contenant le principe actif étalée par élimination du ou des solvant(s), ou solidification du solvant par refroidissement ou par conservation, le cas échéant avec réticulation du polymère, et
d) découpage de pansements individuels à partir de la masse de polymère contenant le principe actif après la solidification.

13. Utilisation d'un dérivé de (-)-morphine et/ou d'un sel d'addition d'acide pharmaceutiquement acceptable d'un dérivé de (-)-morphine pour la fabrication d'un médicament destiné à être utilisé pour l'administration continue et contrôlée du principe actif par un procédé de traitement des symptômes de désintoxication physiques et/ou psychiques liés à l'arrêt brutal d'un abus d'opiacés.

14. Utilisation selon la revendication 13, **caractérisée en ce que** le dérivé de (-)-morphine est la diacétylmorphine.
